# EUROPÄISCHE PATENTSCHRIFT

(11) **EP 0 845 278 B1**
(45) Veröffentlichungstag und Bekanntmachung des Hinweises auf die Patenterteilung: **10.09.2003**
(21) Anmeldenummer: 97120973.9
(22) Anmeldetag: 28.11.1997
(51) Int. Cl.: A61M 25/02

(54) **Befestigungssystem z.B. für ein medizinisches Instrument**
Fastening system for medical instrument
Système de fixation pour instrument médical

(30) Priorität: 26.05.1997 DE 19721963; 28.11.1996 DE 19649430
(43) Veröffentlichungstag der Anmeldung: 03.06.1998
(73) Patentinhaber: Tulafor Anstalt, 9490 Vaduz (LI)
(72) Erfinder: Piwonka, Peter, 4054 Basel (CH)
(74) Vertreter: Körber, Wolfhart, Dr. rer.nat.

(56) Entgegenhaltungen:
- WO-A-86/06641
- US-A- 3 726 280
- US-A- 3 834 380
- US-A- 5 292 312

## Beschreibung

Die vorliegende Erfindung betrifft ein Befestigungssystem zum Befestigen zumindest eines Gegenstandes, beispielsweise eines medizinischen Instrumentes, an einer Oberfläche gemäß dem Oberbegriff des beigefügten Anspruchs 1.

Medizinische Instrumente wie beispielsweise Schläuche oder dergleichen werden herkömmlicher weise durch Pflasterstreifen am menschlichen Körper befestigt. Dabei müssen bei einem Wechsel der medizinischen Instrumente die Pflasterstreifen jedes Mal abgerissen werden, was zu Hautschäden führt. Weiterhin werden die medizinischen Instrumente während der Benutzung häufig hin- und herbewegt, so daß sich die Verbindung zwischen dem Pflasterstreifen und der Haut leicht lösen kann. Ein weiterer Nachteil ist die Verringerung der Haftfähigkeit von üblichen Pflasterstreifen beim Schwitzen oder beim Erhitzen der Haut. Aus den oben genannten Gründen müssen die herkömmlichen Pflasterstreifen häufig gewechselt werden, was zu starken Hautbeeinträchtigungen führen kann.

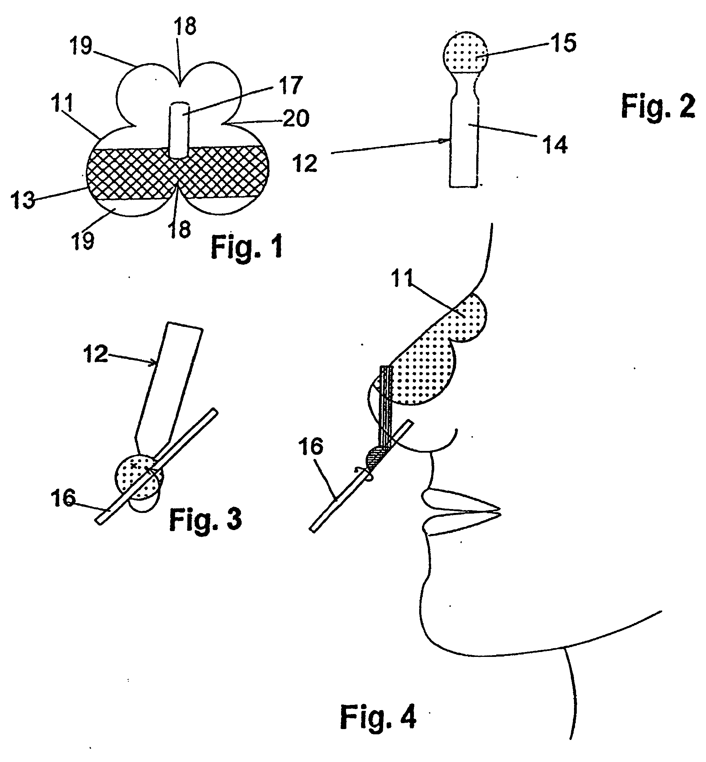

In der internationalen Patentanmeldung W086/06641 ist ein Befestigungssystem zum Befestigen eines medizinischen Instrumentes an einer Oberfläche mittels einem Halteelement gemäß dem Oberbegriff des Anspruchs 1 offenbart. In dem in den Figuren 1 bis 4 der internationalen Anmeldung dargestellten Ausführungsbeispiel umfaßt das Halteelement ein Klettmaterial zum Befestigen des Halteelementes an einem korrespondierenden Klettmaterial der Oberfläche mittels eines Klettverschlusses. Die Oberfläche ist dabei durch ein Pflaster gebildet, das auf der Oberseite ein entsprechendes Klettmaterial zum Befestigen des Halteelementes und auf der Unterseite ein Klebematerial aufweist, damit es beispielsweise an der Haut angeklebt werden kann. Das in der internationalen Anmeldung beschriebene Halteelement ist flexibel und weist eine flächige Form auf, wobei das jeweilige zu haltende medizinische Instrument zwischen dem Halteelement und dem Pflaster eingeklemmt wird. Zu beiden Seiten des eingeklemmten medizinischen Instrumentes wird das Halteelement mittels des Klettverschlusses an dem Pflaster lösbar befestigt.

Die Patentschrift US-A-5 292 312 beschreibt ein ähnliches Befestigungssystem zum Befestigen eines Gegenstandes, beispielsweise eines medizinischen Instrumentes an einer Oberfläche eines Trägerelementes mittels einem Halteelement. D1 offenbart, die Oberfläche sowie das Halteelement mit korrespondierendem Klettmaterial auszustatten, so daß die Oberfläche des Trägerelementes und das Halteelement einen Klettverschluß bilden, und daß ein "klebriges" Material auf dem Halteelement zur Haftung des zu befestigenden Gegenstandes verwendet wird.

Der Nachteil dieser bekannten Befestigungssysteme ist, daß das medizinische Instrument im gehaltenen Zustand zwischen dem Halteelement und dem Pflaster eingeklemmt ist, so daß das Halteelement fest auf das Pflaster gedrückt werden muß, um eine zuverlässige Halterung des medizinischen Instrumentes zu gewährleisten. Es ist jedoch nicht immer möglich, das Halteelement fest auf das Pflaster zu drücken, insbesondere wenn das Pflaster sich beispielsweise auf einer empfindlichen Körperpartie befindet. Weiterhin müssen die Form des Halteelementes und die Form des Pflasters jeweils speziell aufeinander und auf die Form des zu haltenden medizinischen Instrumentes abgestimmt sein, da das medizinische Instrument durch die Befestigung des Halteelementes auf dem Pflaster gehalten wird. Damit ist für jedes unterschiedliche medizinische Instrument ein speziell ausgestaltetes Befestigungssystem notwendig.

Ein weiterer Nachteil ist, daß beim Auswechseln des gehaltenen medizinischen Instrumentes das Halteelement und das auszuwechselnde medizinische Instrument gleichzeitig ergriffen werden müssen, da beim Lösen des Halteelementes von dem Pflaster das medizinische Element sofort freigegeben ist. Das ist besonders bei der Verwendung des Befestigungssystems in Krankenhäusern oder dergleichen unpraktisch, da dort oft jede freie Hand dringend benötigt wird.

Die Aufgabe der vorliegenden Erfindung besteht somit darin, ein Befestigungssystem gemäß dem Oberbegriff des beigefügten Anspruches 1 bereitzustellen, mit dem unterschiedliche Gegenstände, wie z.B. medizinische Instrumente, einfach und zuverlässig an einer Oberfläche befestigt und gehalten werden können, wobei gleichzeitig eine ausreichende Bewegungsfreiheit sowie eine einfache Auswechselbarkeit des gehaltenen Gegenstandes bzw. Instrumentes gewährleistet ist.

Diese Aufgabe wird durch ein Befestigungssystem mit den Merkmalen des beigefügten Anspruches 1 gelöst.

Im Unterschied zu dem oben beschriebenen Stand der Technik weist das Halteelement an seinem ersten Ende das Klettmaterial zum Befestigen des Halteelementes an der Oberfläche auf und an dem zweiten Ende zumindest auf einer Seite eine Haftschicht zum Halten des zumindest einen zu fixierenden Gegenstand, so daß der zumindest eine Gegenstand während der Befestigung des Halteelementes an der Oberfläche des Trägerelementes durch zumindest einen Oberflächenabschnitt des Trägerelementes gebildet ist, das auf seiner dem Oberflächenabschnitt abgewandten Seite ein Klebematerial aufweist.

Hierdurch wird eine zusätzliche Zugentlastung und Bewegungsfreiheit des gehaltenen Gegenstandes bzw. medizinischen Instrumentes gewährleistet. Wird der gehaltene Gegenstand bzw. das medizinische Instrument während der Benutzung bewegt oder verschoben, so wird die Bewegung von dem Halteelement aufgenommen, in dem der Gegenstand bzw. das medizinische Instrument gehalten ist, und zusätzlich von der Klettverbindung zwischen dem Halteelement und der Oberfläche. Die vorliegende Erfindung ist besonders vorteilhaft in Krankenhäusern u.ä. zu verwenden, da dort einfache, mit der Hand zu bedienende und zuverlässig funktionierende Befestigungssysteme zur Befestigung von medizinischen Instrumenten an Patienten oder an sonstigen Oberflächen notwendig sind. Die vorliegende Erfindung wird deshalb im Folgenden insbesondere in Bezug auf die Befestigung medizinischer Instrumente erläutert.

Das erfindungsgemäße Halteelement, in dem ein medizinisches Instrument gehalten ist, kann weiterhin zum Auswechseln des medizinischen Instruments einfach mit einer Hand von der Oberfläche gelöst werden, ohne dass gleichzeitig das medizinische Instrument mit der anderen Hand ergriffen werden muss. Damit kann die Lage von medizinischen Instrumenten auf einfache Weise korrigiert werden, indem lediglich der Klettverschluss zwischen dem Halteelement und der Oberfläche gelöst und in veränderter Lage wieder hergestellt wird.

Ein weiterer Vorteil des erfindungsgemäßen Befestigungssystems ist seine universelle Verwendbarkeit mit verschiedenen Arten medizinischer Instrumente. Da der Klettverschluß zwischen dem Halteelement und der Oberfläche unabhängig von der Form des medizinischen Instruments ist, das ausschließlich in dem Halteelement gehalten ist, können unterschiedliche medizinische Instrumente auf flexible Weise befestigt werden.

Vorteilhafte Ausgestaltungen der vorliegenden Erfindung sind in den Unteransprüchen angegeben.

Das Halteelement wird erfindungsgemäß auf dem flächigen flexiblen Trägerelement befestigt, das beispielsweise auf der menschlichen Haut aufgeklebt sein kann. Auf diese Weise können die unterschiedlichsten medizinischen Instrumente am menschlichen Körper fixiert werde, wie z. B. Leitungen, Schläuche, Katheter, Sonden, Drainagen, Transducer, Monitoring-Teile, Abzweigungen usw., ohne die Haut zu beeinträchtigen oder empfindliche Stellen einem zu starken Druck auszusetzen. Da das bzw. die zu haltenden medizinischen Instrumente bereits in dem Halteelement gehalten sind, muss das Halteelement beim Befestigen nur leicht auf das auf der Haut klebende Trägerelement aufgedrückt werden, um es zu befestigen. Das ist insbesondere dann von Vorteil, wenn das Trägerelement sehr lange an einer Stelle kleben muss. Ein derart befestigtes medizinisches Instrument kann weiterhin auf einfache Weise mit einer Hand vom auf der Haut klebenden Trägerelement gelöst, in seiner Lage verändert oder ausgetauscht werden.

Die Form des flächigen flexiblen Trägerelementes ist in einer vorteilhaften Ausgestaltung an eine menschliche Nase angepaßt, so daß es auf eine Nase aufgeklebt werden kann. Diese Ausgestaltung ist insbesondere zur sicheren und einfachen Befestigung von Nasensonden geeignet, die in dem erfindungsgemäßen Halteelement gehalten werden. Durch die einerseits eine zuverlässige Halterung, andererseits einen Bewegungsspielraum der Nasensonde ermöglichende Ausgestaltung und Befestigung des Halteelementes auf die Oberfläche des Trägerelementes wird sichergestellt, daß eine Nasensonde weder zu tief in die Nase eintreten noch aus ihr austreten kann.

Vorteilhafterweise ist das Klebematerial des Trägerelements ein Hydrocolloid. Dieses Material ist auf Naturbasis gefertigt und erzeugt keine Hautreizungen oder Allergien. Darüber hinaus kann ein Hydrocolloid lange Zeit auf der Haut verbleiben, da es die Hautfeuchtigkeit aufnimmt und auch auf schwitzender Haut klebt. Die Verwendung eines Hydrocolloids als Klebematerial des Tägerelements ist damit insbesondere bei der Befestigung von medizinischen Instrumenten an der Haut kranker Menschen von Vorteil, da diese häufig stark schwitzen bzw. eine feuchte Haut haben. Normale und selbst hochwertige Pflaster kleben auf schwitzender Haut nicht mehr.

Die Oberfläche, an der das Halteelement mittels des Klettverschlusses lösbar befestigt werden kann, kann die Oberfläche einer beispielsweise medizinischen Vorrichtung sein, die als Flauschmaterial für den Klettverschluß geeignet ist. Das Klettmaterial des Halteelements muss in diesem Fall das entsprechende Haftmaterial für den Klettverschluß sein. Das Halteelement gemäß der vorliegenden Erfindung ist somit vielseitig einsetzbar und kann nicht nur an den entsprechenden flexiblen flächigen Trägerelementen, sondern auch an geeigneten Oberflächen von Vorrichtungen befestigt werden.

Die vorliegende Erfindung wird im folgenden anhand bevorzugter Ausführungsbeispiele unter Bezug auf die beigefügten Zeichnungen näher erläutert, in denen zeigen
Figur 1 eine Draufsicht auf eine erste Ausgestaltung eines möglichen herkömmlichen Trägerelementes;
Figur 2 eine Draufsicht auf eine erste Ausgestaltung des Halteelementes gemäß der vorliegenden Erfindung;
Figur 3 eine Draufsicht auf das in Figur 2 gezeigte Halteelement mit einem zu haltenden medizinischen Instrument;
Figur 4 eine Seitenansicht des an einer Nase befestigten Trägerelementes von Figur 1 an dem ein eine Nasensonde haltendes Halteelement gemäß der Figuren 2 und 3 befestigt ist;
Figur 5 eine Draufsicht auf eine zweite Ausgestaltungdes Trägerelementes gemäß der vorliegenden Erfindung;
Figur 6 eine Draufsicht auf eine zweite Ausgestaltung des Halteelementes gemäß der vorliegenden Erfindung;
Figur 7 eine Unteransicht des in Figur 6 gezeigten Halteelementes;
Figur 8 eine Seitenansicht des in Figur 6 gezeigten Halteelementes;

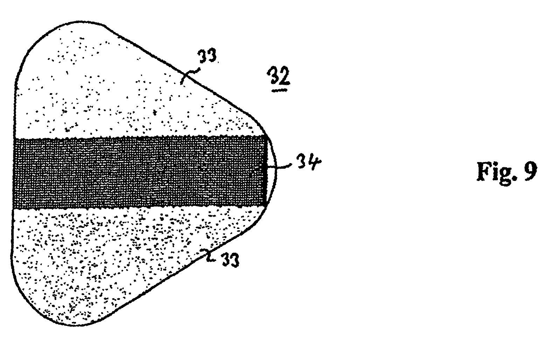

Figur 9 eine Draufsicht auf eine dritte Ausgestaltung des Halteelementes gemäß der vorliegenden Erfindung.

In Figur 1 ist eine Draufsicht auf eine erste Ausgestaltung eines Trägerelementes 11 gemäß der vorliegenden Erfindung dargestellt. Das Trägerelement ist an die Form einer menschlichen Nase angepaßt und kann auf eine Nase aufgeklebt werden, wie in Figur 4 gezeigt ist. Das Trägerelement 11 ist als Pflaster ausgebildet, an dessen Unterseite sich eine Klebeschicht zum Ankleben des Pflasters an der Nase eines Menschen befindet. Im Bereich seines distalen Endes hat das Trägerelement 11 einen querverlaufenden Oberflächenabschnitt 13 aus Klettmaterial, das einen Teil des Klettverschlusses mit einem entsprechenden Halteelement verbindet und beispielsweise mit einem Haftmaterial für den Klettverschluß versehen ist. Das schmetterlingsförmig ausgebildete Trägerelement 11 ist an den Ecken 19 abgerundet und weist an seinem distalen und an seinem proximalen Ende in der Mitte jeweils einen Einschnitt 18 auf. Entsprechende Einschnitte 20 sind auch seitlich vorhanden. Im Mittelbereich des Trägerelementes 11, der direkt auf den Nasenrücken aufgeklebt wird, ist ein Ausschnitt 17 vorgesehen. Die Breite des Trägerelementes 11 ist dabei dergestalt, daß die Nase ausreichend überdeckt werden kann, um eine ausreichende Haftung des Trägerelementes 11 auf der Nase sicherzustellen. Weiterhin nimmt die Breite des Trägerelementes 11 von seinem distalen zu seinem proximalen Ende hin ab.

In Figur 2 ist eine Draufsicht einer ersten Ausgestaltung eines Halteelementes 12 gemäß der vorliegenden Erfindung dargestellt. Das Halteelement 12 ist aus flexiblem Material und bandförmig ausgebildet. Es weist einen rechteckigen länglichen Endabschnitt 14 und einen runden Endabschnitt 15 auf. Der rechteckige Endabschnitt 14 bildet den entsprechenden beispielsweise mit dem Trägerelement 11 zu verbindenden anderen Teil des Klettverschlusses und kann daher beispielsweise aus einem Flauschmaterial bestehen. Der verbreiterte runde Endabschnitt 15 am distalen Ende des Halteelementes 12 weist eine Klebeschicht auf, an der ein medizinisches Instrument wie z.B. eine Nasensonde befestigt werden kann.

In Figur 3 ist das in Figur 2 dargestellte Halteelement gemeinsam mit einem an der Klebeschicht zu befestigenden medizinischen Instrument in Form einer Nasensonde 16 dargestellt. Die Nasensonde 16 ist ein dünner Schlauch, der auf das das Klebematerial aufweisende verbreiterte Ende 15 des Halteelementes 12 gelegt und festgeklebt wird. Die Nasensonde 16 wird dabei schräg auf das verbreiterte distale Ende 15 des Halteelementes 12 gelegt, das dann über die Nasensonde 16 geklappt wird. Das Halteelement 12 ist zwischen dem verbreiterten distalen Ende 15 und dem rechteckigen Endabschnitt 14 verengt, um eine bessere Befestigung der Nasensonde 16 zu ermöglichen.

In Figur 4 ist in einer Seitenansicht dargestellt, wie das gesamte in den Figuren 1-3 erläuterte Befestigungssystem an einer Nase befestigt wird. Das Trägerelement 11 wird dergestalt auf die Nase geklebt, das sein breiteres Ende in Richtung der Nasenspitze zeigt. Das breitere distale Ende weist dabei einen querliegenden Abschnitt 13 mit dem Klettmaterial auf, an dem das Halteelement 12 mit seinem rechteckigen Endabschnitt 14 befestigt wird, der das korrespondierende Klettmaterial aufweist. Der Abschnitt 13 kann dabei ein Haftmaterial für den Klettverschluß aufweisen, während der rechteckige Endabschnitt 14 das entsprechende Flauschmaterial für den Klettverschluß aufweist, oder auch umgekehrt. Das Trägerelement 12 hängt, wie in Figur 4 dargestellt ist, von der Nase herunter und hält die Nasensonde 16 in der Nase, wobei die Nasensonde einen Spielraum hat, da das Trägerelement 12 aus flexiblem Material besteht. Durch diesen Spielraum der Nasensonde 16 wird sichergestellt, daß der Patient die Nasensonde nicht verliert, wenn er sich bewegt, wobei durch das erfindungsgemäße Befestigungssystem trotzdem eine sichere und zuverlässige Befestigung der Nasensonde gewährleistet ist. Das Halteelement 12 kann abhängig von der Lage des Patienten oder sonstigen Erfordernissen an einer beliebigen Stelle des querverlaufenden Abschnittes 13 des Trägerelementes befestigt werden, so daß die Nasensonde 16 entweder in das eine oder das andere Nasenloch eingeführt werden kann.

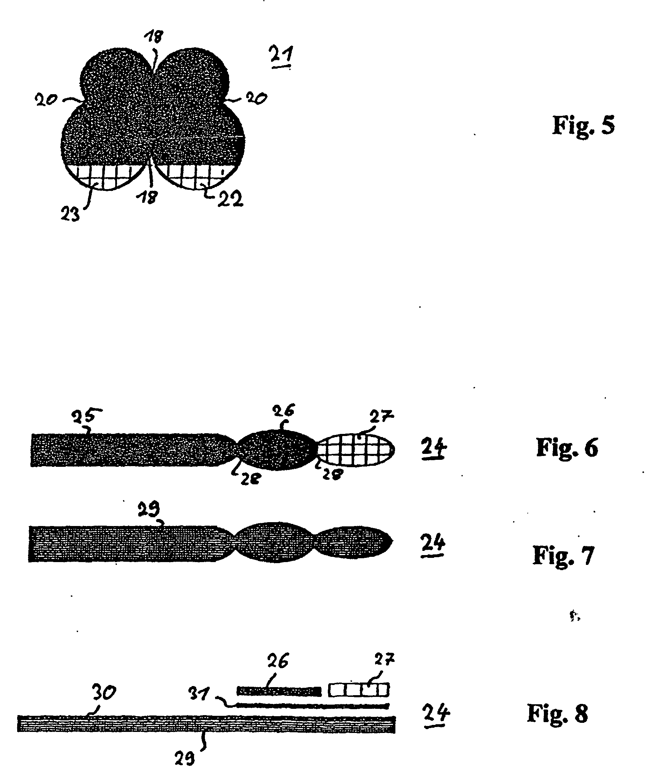

In Figur 5 ist eine Draufsicht auf eine zweite Ausgestaltung eines Trägerelementes 21 gemäß der vorliegenden Erfindung gezeigt. Die Form des Trägerelementes 21 entspricht im wesentlichen der Form des in Figur 1 gezeigten Trägerelementes, jedoch weist das Trägerelement 21 keine Ausnehmung auf. Ein weiterer Unterschied ist, daß das Trägerelement 21 keinen querliegenden Abschnitt 13 mit Klettmaterial, sondern zwei getrennte Abschnitte 22 und 23 an den beiden distalen Enden des Trägerelementes 21 aufweist. Die beiden Endabschnitte 22 und 23 mit Klettmaterial sind durch einen Einschnitt 18 in Längsrichtung voneinander getrennt. Ein ebensolcher Einschnitt 18 ist auch auf der anderen Seite des Trägerelementes vorhanden. Ebenso wie das Trägerelement 11 weist auch das Trägerelement 21 seitliche Einschnitte 20 auf.

In Figur 6 ist die Draufsicht auf ein Halteelement 24 gemäß der vorliegenden Erfindung dargestellt. Das Halteelement 24 besteht aus einem flexiblen Material und ist bandförmig ausgebildet. Es umfaßt einen rechteckigen Endabschnitt 25, einen abgerundeten Mittelabschnitt 26 und einen abgerundeten Endabschnitt 27, der dem Endabschnitt 25 gegenüberliegt. Zwischen dem Endabschnitt 25 und dem Mittelabschnitt 26, sowie zwischen dem Mittelabschnitt 26 und dem Endabschnitt 27 weist das Halteelement 24 Einschnitte 28 auf. Der rechteckige Endabschnitt 25 besteht aus einem Basismaterial und der abgerundete Endabschnitt 27 besteht aus einem Klettmaterial, so daß der Endabschnitt 27 und die Unterseite 29 zusammen einen Klettverschluß bilden können. Dabei kann die Unterseite 29 beispielsweise das Flauschmaterial des Klettverschlusses aufweisen, während der Endabschnitt 27 das Haftmaterial umfaßt, oder umgekehrt. Das in dem Halteelement 24 zu haltende medizinische Instrument wird, wenn der Endabschnitt 27 an dem Endabschnitt 25 befestigt ist, durch den Mittelabschnitt 26, der dabei eine Schlaufe bildet, gehalten. Vorteilhafterweise weist der Mittelabschnitt 26 dabei eine Schicht aus einem rutschhemmenden Material oder sogar ein Klebematerial auf, so daß ein zuverlässiges Halten des medizinischen Instrumentes gewährleistet ist.

In Figur 7 ist eine Unteransicht des in Figur 6 gezeigten Halteelementes 24 dargestellt. Die gesamte Unterseite 29 des Halteelementes 24 ist durch das Flauschband eines Klettverschlusses gebildet und kann daher auch zum Befestigen an den entsprechenden Stellen eines Trägerelementes verwendet werden. Der Aufbau des Halteelementes 24 ist in Figur 8 verdeutlicht, die eine Seitenansicht des Halteelementes 24 zeigt. Das Halteelement 24 besteht im wesentlichen aus dem Flauschband 29, das auf seiner Oberseite durch die entsprechende Flauschbandoberseite 30 abgeschlossen ist. An einem Ende des Halteelementes 24 wird mittels eines Transferklebstoffes 31 auf dem Mittelabschnitt 26 eine Klebeschicht befestigt, während auf dem Endabschnitt 27 das Haftmaterial des Klettverschlusses aufgeklebt wird. Zum Halten eines medizinischen Instrumentes wie z.B. eines Schlauches wird der Schlauch quer zum Halteelement 24 auf den Mittelabschnitt 26 gelegt, woraufhin der Endabschnitt 27 unter Bildung einer Schlaufe und unter Verdrehung des Halteelementes 24 mit dem Flauschband 29 mit der Unterseite 29 verbunden wird.

## Patentansprüche

1. Befestigungssystem zum Befestigen zumindest eines Gegenstandes (16), beispielsweise eines medizinischen Instrumentes, an einer Oberfläche mit einem Trägerelement (11,21,32), wobei das Trägerelement (11,21,32) zumindest einen Oberflächenabschnitt (11,22,34) und auf seiner dem Oberflächenabschnitt abgewandten Seite ein Klebematerial aufweist, und mit einem Halteelement (12, 24) zum Halten des zumindest einen Gegenstandes an dem Oberflächenabschnitt des Trägerelementes (11, 21, 32), wobei das Halteelement (12, 24) aus einem flexiblen Material besteht, bandförmig ist und ein erstes und ein zweites Ende aufweist und wobei der Oberflächenabschnitt und das Halteelement (12, 24) ein Klettmaterial zum Befestigen des Halteelementes (12, 24) an dem Oberflächenabschnitt aufweisen und wobei die beiden Klettmaterialien korrespondieren und somit einen Klettverschluß bilden,
**dadurch gekennzeichnet,**
**daß** das Halteelement an dem ersten Ende das Klettmaterial zum Befestigen des Halteelementes an dem Oberflächenabschnitt und an dem zweiten Ende zumindest auf einer Seite eine Haftschicht zum Halten des zumindest einen Gegenstandes aufweist, an der der zumindest eine Gegenstand fixierbar ist, so daß der zumindest eine Gegenstand während der Befestigung des Halteelementes an dem Oberflächenabschnitt des Trägerelementes ausschließlich in bzw. an dem Halteelement gehalten ist.

2. Befestigungssystem zum Befestigen zumindest eines Gegenstandes (16), beispielsweise eines medizinischen Instrumentes, an einer nach Anspruch 1,
**dadurch gekennzeichnet,**
**daß** das flächige Trägerelement (11, 21) eine an eine menschliche Nase angepaßte Form aufweist, so daß es auf eine Nase aufgeklebt werden kann.

3. Befestigungssystem zum Befestigen zumindest eines Gegenstandes (16), beispielsweise eines medizinischen Instrumentes, an einer Oberfläche nach Anspruch 1,
**dadurch gekennzeichnet,**
**daß** das Klebematerial des Trägerelements ein Hydrocolloid ist.

## Claims

1. Fastening system for fastening at least one object (16), for example, a medical instrument, to a surface, with a carrier element (11, 21, 32), wherein the carrier element (11, 21, 32) provides at least one surface region (11, 22, 34) and an adhesive material on the side opposing the surface region, and with a holding element (12, 24) for holding the at least one object onto the surface region of the carrier element (11, 21, 32), wherein the holding element (12, 24) is made of a flexible material in a strip shape and provides a first and a second end, wherein the surface region and the holding element (12, 24) provide a burred material for fastening the holding element (12, 24) to the surface region, and wherein the two burred materials are matched to one another to form a Velcro-type fastening,
**characterised in that**,
the holding element provides the burred material, for fastening the holding element onto the surface region, at its first end, and an adhesive layer, for holding the at least one object, on at least one side of its second end, to which adhesive layer the at least one object can be fastened, so that the at least one object is held exclusively in and/or on the holding element while the holding element is fastened to the surface region of the carrier element.

2. Fastening system according to claim 1 for fastening at least one object (16), for example, a medical instrument, to a surface,
**characterised in that**,
the flat carrier element (11, 21) provides a shape adapted to the human nose, so that the carrier element can be fastened to a nose.

3. Fastening system according to claim 1 for fastening at least one object (16), for example, a medical instrument, to a surface,
**characterised in that**,
the adhesive material of the carrier element is a hydrocolloid.

## Revendications

1. Système de fixation pour fixer au moins un objet (16), par exemple un instrument médical, à une surface avec un élément de support (11, 21, 32), où l'élément de support (11, 21, 32) présente au moins un tronçon de surface (11, 22, 34) et sur son côté éloigné du tronçon de surface un matériau collant, et avec un élément de retenue (12, 24) pour retenir l'objet précité au tronçon de surface de l'élément de support (11, 21, 32), où l'élément de retenue (12, 24) est réalisé en un matériau flexible, est en forme de bande et présente une première et une seconde extrémité, et où le tronçon de surface et l'élément de retenue (12, 24) présentent un matériau auto-accrochant pour la fixation de l'élément de retenue (12, 24) au tronçon de surface, et où les deux matériaux auto-accrochants correspondent et forment ainsi une fermeture auto-accrochante,
**caractérisé**
**en ce que** l'élément de retenue présente à la première extrémité le matériau auto-accrochant pour la fixation de l'élément de retenue au tronçon de surface et à la seconde extrémité au moins sur un côté une couche adhésive pour retenir au moins un objet précité, à laquelle au moins un objet précité peut être fixé de telle sorte qu'au moins un objet précité, pendant la fixation de l'élément de retenue au tronçon de surface de l'élément de support, est retenu exclusivement dans respectivement à l'élément de retenue.

2. Système de fixation pour la fixation d'au moins un objet (16), par exemple d'un instrument médical, à une surface selon la revendication 1,
**caractérisé**
**en ce que** l'élément de support plan (11, 21) présente une forme adaptée au nez humain de sorte qu'il peut être collé sur un nez.

3. Système de fixation pour la fixation d'au moins un objet (16), par exemple d'un instrument médical, à une surface selon la revendication 1,
**caractérisé**
**en ce que** le matériau collant de l'élément de support est un hydrocolloïde.
